# EUROPEAN PATENT SPECIFICATION

(11) **EP 3 301 185 B1**
(45) Date of publication and mention of the grant of the patent: **15.09.2021**
(21) Application number: 15907079.6
(22) Date of filing: 16.12.2015
(51) Int. Cl.: C12Q 1/68, C12Q 1/04, C12R 1/01, C12Q 1/6883

(54) **INTESTINAL METAGENOMIC FEATURE AS SELECTION MARKER OF CURATIVE EFFECT OF ACARBOSE FOR TREATING TYPE 2 DIABETES**
INTESTINALES METAGENOMES FEATURE ALS SELEKTIONSMARKER DER HEILENDEN WIRKUNG VON ACARBOSE ZUR BEHANDLUNG VON TYP-2-DIABETES
CARACTÉRISTIQUE MÉTAGÉNOMIQUE INTESTINALE UTILISÉE COMME MARQUEUR DE SÉLECTION DE L'EFFET CURATIF DE L'ACARBOSE POUR TRAITER LE DIABÈTE DE TYPE 2

(30) Priority: 26.10.2015 CN 201510703463
(43) Date of publication of application: 04.04.2018
(73) Proprietor: Shanghai Institute for Endocrine and Metabolic Diseases, Shanghai 200025 (CN); BGI Shenzhen, Shenzhen, Guangdong 518000 (CN)
(72) Inventor: NING, Guang, Shanghai 200025 (CN); ZHANG, Dongya, Shenzhen Guangdong 518000 (CN); WANG, Weiqing, Shanghai 200025 (CN); WANG, Xiaokai, Shenzhen Guangdong 518000 (CN); GU, Yanyun, Shanghai 200025 (CN); FENG, Qiang, Shenzhen Guangdong 518000 (CN); LIU, Ruixin, Shanghai 200025 (CN); XU, Xiaoqiang, Shenzhen Guangdong 518000 (CN)
(74) Representative: Sun, Yiming
(86) International application number: PCT/CN2015/097566
(87) International publication number: WO 2017/071018

(56) References cited:
- WO-A1-2014/019408
- WO-A1-2014/053608
- WO-A1-2014/060538
- WO-A2-2008/076696
- WO-A2-2008/076696
- CN-A- 102 477 460
- CN-A- 104 540 962
- CN-A- 104 603 283
- CN-A- 104 726 596
- SAMA?I. SAYIN ET AL: "Gut Microbiota Regulates Bile Acid Metabolism by Reducing the Levels of Tauro-beta-muricholic Acid, a Naturally Occurring FXR Antagonist", CELL METABOLISM, vol. 17, no. 2, 1 February 2013 (2013-02-01), pages 225-235, XP055145995, ISSN: 1550-4131, DOI: 10.1016/j.cmet.2013.01.003
- SI , WEN: 'Effects of Acarbose on Serums IL -4, IL -6 and IL -10 of Patients with Type 2 Diabetes' CHINA MASTER'S S THESES FULL-TEXT DATABASE 15 June 2012, pages 5 - 7, XP009502949
- None

## Description

### Technical Field

The present invention relates to an application of characteristics of gut microbiota metagenome as a screening marker of Acarbose efficacy in patients with Type 2 diabetes.

### Background Art

At present, drug efficacy judgment and pre-treatment classification diagnosis are not available in the treatment of Type 2 diabetes. The pathological and physiological mechanism of Type 2 diabetes is mainly insulin resistance and insulin secretion deficiency. Although there are drugs for insulin resistance and insulin secretion deficiency for the treatment of Type 2 diabetes, no scientific and feasible method for the classification diagnosis based on insulin resistance or insulin secretion deficiency is available.

Currently the clinically feasible program is to measure the patient BMI, waist circumference and insulin level. According to the BMI, waist circumference that exceed Chinese standard, or the HOMAIR calculated by patient's fasting blood glucose or insulin level, the insulin resistance can be judged. There are no universal standard for the insulin level at home and abroad, generally it is represented by the HOMA β index calculated by patient's blood glucose and insulin, but it cannot be used as an index for determining the degree of insulin resistance and insulin secretion deficiency, therefore it is unable to meet the requirements for precision medical care.

Clinically, the glucose clamp test is used to accurately assess the insulin resistance and β cell functions. The glucose clamp test with positive-glucose high insulin level is used to assess the insulin resistance levels, while the clamp test with high glucose level is used to assess the β cell insulin secretion functions. The two methods take long time and patients need to lie in bed for 4 to 5 hours. The operations must be completed by experienced nurses. Blood should be collected from multiple points for the real-time monitoring of blood glucose and the determination of insulin level. This method is expensive, with poor patient compliance, so it is difficult to be carried out clinically.

The precision medicine raises the requirement of individualized diagnosis and treatment. The tumor-targeted drugs have been used clinically. However, no effective regimen for targeted therapy of Type 2 diabetes has been found so far. There are a number of therapeutic regimens for the Type 2 diabetes and patients have different response, which lead to a low blood glucose control rate for patients with Type 2 diabetes. For the main pathogenesis of Type 2 diabetes, there are a variety of drugs for insulin secretion deficiency and insulin resistance, but no simple and exact clinical diagnosis method for insulin secretion deficiency or insulin resistance is available.

The existing studies use the liver, fat (insulin resistance) and islet β cells (islet function) as the main organs involved in Type 2 diabetes. Recently, the pathological and physiological functions of gut microbiota and intestinal mucosal epithelial absorption, barrier and endocrine are increasingly recognized for the pathogenesis of Type 2 diabetes and treatment strategy. For example, gut microbiota metagenome studied have shown that there was significant difference in the gut microbiota between patients with Type 2 diabetes and normal patients [Qin, J., et al., A metagenome-wide association study of gut microbiota in Type 2 diabetes. Nature, 2012.]. The gut-modified bariatric surgery could reduce the body weight of obese patients, and surprisingly, the blood glucose in obese patients with Type 2 diabetes was well controlled without medication after surgery, and even cured completely [Carlsson, L.M.S., et al., Bariatric Surgery and Prevention of Type 2 Diabetes in Swedish Obese Subjects. New England Journal of Medicine, 2012. 367(8): p. 695-704, Schauer, P.R., et al., Bariatric surgery versus intensive medical therapy for diabetes--3-year outcomes. N Engl J Med, 2014. 370(21): p. 2002-13]. The drugs that simulate intestinal hormones, such as GLP-1 agonists and DPPIV inhibitors, have become oral hypoglycemic agents with highest prescription dose in the world, and related cardiovascular benefits have been reported.

The concept of enterotype [Arumugam, M., et al., Enterotypes of the human gut microbiome. Nature, 2011. 473 (7346): p. 174-80] was first proposed by Peer Bork, which meant that the composition of gut microbiota was relatively fixed in the populations. There are 2 to 3 kinds of enterotypes in the populations. With the increased sample size and the improved sequencing technique, especially the promotion of the second generation of sequencing, the enterotype can be classified into 2 types: one is the Prevotella-based Prevotella enterotype, and the other is Bacteroides -based Bacteroides enterotype. At present, no evidence has shown the direct association between enterotype and various medical health indexes of human body. The corresponding gene function studies suggest the metabolic ability of vitamins is varied for different enterotypes, which is associated with the meat-vegetable dietary habit of the host. However, although gut microbiota is also considered to be an important medium of metabolism in human body [Haiser, H.J. and P.J. Turnbaugh, Is it time for a metagenomic basis of therapeutics? Science, 2012. 336(6086): p. 1253-5], no clinical trial evidences can be available now.

### Summary of Invention

An object of the present invention is to overcome the drawback of lack of directly relevant evidences between the enterotypes and health indicators of the human body in the prior art and provide characteristics of gut metagenome as a screening marker of Acarbose efficacy in patients with Type 2 diabetes; particularly provide a use of characteristics of gut microbiota metagenome as a screening marker of Acarbose efficacy in patients with Type 2 diabetes. In the present invention, it is discovered that patients with Type 2 diabetes with different gut microbiota showed significant difference in the therapeutic response to diabetic hypoglycemic agent-Acarbose. Therefore, the Bacteroides-based Bacteroides enterotype can be used as a screening marker of Acarbose efficacy in patients with Type 2 diabetes.

The object of the present invention is achieved through the following technical solutions:
The present invention relates to a use of characteristics of gut microbiota metagenome as a screening marker of Acarbose efficacy in patients with Type 2 diabetes, wherein the characteristics of gut microbiota metagenome is Bacteroides enterotype.

Preferably, the Bacteroides enterotype is determined by DNA sequencing or PCR amplification of gut microbiota in feces ex vivo.

Preferably, the PCR amplification specifically comprises: extract the DNA of gut microbiota in feces ex vivo and perform 16Srna PCR amplification for specific enrichment strains.

Preferably, the Bacteroides enterotype is determined by detecting secondary bile acid in the ex vivo blood samples. The secondary bile acids include UDCA, TUDCA, GUDCA, DCA, TDCA, GDCA, LCA, TLCA, GLCA. In the present invention, two kinds of enterotypes are found, one is Prevotella-based Prevotella enterotype, and the other is Bacteroides -based Bacteroides enterotype. In the Bacteroides enterotype, the deoxycholic acid and lithocholic acid levels are significantly lower than those in Prevotella enterotype, while the ursodeoxycholic acid level with protective effect is higher than that in the Prevotella enterotype. The further gut metagenome analysis showed that, ursodesoxycholic acid is further decomposed into KO of lithocholic acid, which is apparently enriched in the Prevotella enterotype, suggesting that the metabolic ability of bile acids in gut microbiota was significantly different in patients with two enterotypes.

Preferably, the detection of secondary bile acid comprises the following steps:
S1. Sample pretreatment: Add 300 µL of internal standard methanol to every 75 µL of blood samples, to extract the target compound and precipitate the protein, vortex, centrifuge and draw the supernatant, then lyophilize, re-dissolve in 50 µL of acetonitrile solution (25%, volume), and wait for sample injection;
S2. Detection: conduct sample analysis using 1290 Infinity liquid phase and 6460A triple quadrupole mass spectrometry system;

Perform the liquid phase separation using 100mm×2.1mm ACQUITY UPLC C8 column having a particle size of 1.7 m, of which, phase A is 10 mM NH4HCO3 aqueous solution, phase B is pure acetonitrile; initially 25% phase B (by volume), retaining 0.5 min, followed by increased to 40% phase B (by volume) linearly within 12.5 min, then increased to 90% (by volume) within 1 min, flush the system for 3min, recover to 25% phase B (by volume) in 0.5 min, after equilibrating 2.5 min, the flow rate is 0.35 ml/min, column temperature is 35°C and the injection volume is 5 µL;

Mass spectrometry is performed by ESI source negative ion mode, with main parameters as follows: Gas Temp: 350°C; Gas Flow: 8 I/min; Nebulizer: 40 psi; Sheath Gas Temp: 400 °C; Sheath Gas Flow: 8 I/min; Capillary: 3500 V; Nozzle voltage: 400 V.

Preferably, the efficacy of Acarbose in the patients with Type 2 diabetes and Bacteroides enterotype includes improving the insulin resistance, reducing the secondary bile acid, and promoting the reduction of cardiovascular risks in addition to glucose-lowering.

Preferably, the indicators for reducing the harmful secondary bile acid include GDCA, TDCA, TLCA, and the indicators for reducing the taurine-conjugated bile acid include TCA, TDCA, TLCA, TUDCA.

Preferably, the indicators for improving insulin resistance include decreased fasting blood glucose, decreased fasting C peptide and insulin level, down-regulated waist-to-hip ratio, down-regulated HOMA insulin resistance index and up-regulated Adiponectin.

Preferably, the indicators that promote the reduction of cardiovascular risks include decreased PDGFAA, PDGFAABB, endothelin, and VegfC plasma factor.

Also described herein is a kit used for screening of Acarbose efficacy in patients with Type 2 diabetes, comprising:
A reagent used to collect ex vivo stool samples or ex vivo blood samples;
A reagent used to determine the enterotype by DNA sequencing or PCR amplification of the gut microbiota in the ex vivo stool samples, or a reagent used to determine the enterotype by detecting the secondary bile acid in the ex vivo blood samples.

There are two kinds of enterotypes, one is Prevotella-based Prevotella enterotype, and the other is Bacteroides -based Bacteroides enterotype. Different enterotype can predict the benefits of patients for treatment of diabetes with Acarbose, especially the effect of improving insulin resistance, reducing secondary bile acid, and promoting the reduction of cardiovascular risks in addition to glucose-lowering. Specifically, Bacteroides enterotype has a better effect of improving insulin resistance, reducing secondary bile acid, and promoting the reduction of cardiovascular risks in addition to glucose-lowering.

Compared with prior art, the prevent invention can achieve the following beneficial effects:
1) It is discovered that patients with Type 2 diabetes with different gut microbiota showed significant difference in the therapeutic response to diabetic hypoglycemic agent-Acarbose by using the concept of enterotype firstly. Therefore, before medication, patients can be classified according to the enterotype, to select the populations with optimal efficacy and determine if an individual patient with Type 2 diabetes is applicable to Acarbose treatment.
2) The classification of enterotypes is generally based on DNA sequencing or PCR amplification of gut microbiota in the feces; while in the baseline, the bile acid component, especially secondary bile acid, can be used for distinguishing the enterotypes; the typing of gut microbiota (i.e. enterotype) can be identified by the blood markers (i.e. secondary bile acid level in the plasma), to become a marker for diagnosis.

### Brief Description of the Drawings

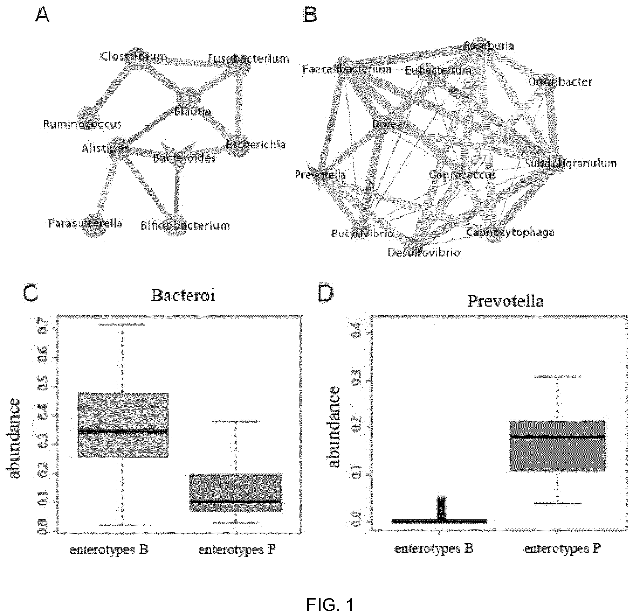

FIG. 1 is a schematic diagram of the typing of the enterotypes, of which, A. horizontal clustering and correlation of Bacteroides enterotype; B. horizontal clustering and correlation of Prevotella enterotype; C. Comparison of Bacteroides biological abundance in two enterotypes; D. Comparison of Prevotella biological abundance in two enterotypes. EntB: Bacteroides enterotype. EntP: Prevotella enterotype;

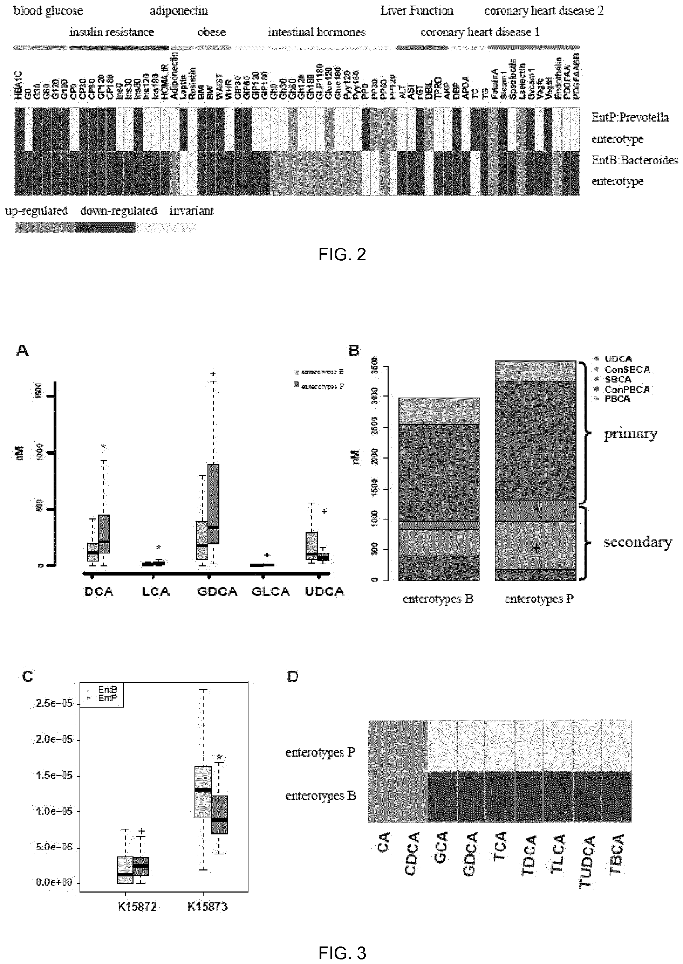

FIG.2 shows the changes in curative effect indexes after treatment with Acarbose in two enterotypes, the black represents a significant decrease after treatment, white represents a significant increase after treatment, and gray represents no significant change after treatment, P <0.05;
FIG. 3 shows the difference of bile acid spectra and their changes after Acarbose treatment between the two enterotypes, of which, A. bile acids with difference in the baseline in two enterotypes; B. Difference of bile acid spectra between two enterotypes at baseline. C. Difference of two KO related to secondary bile acid metabolism between two enterotypes at baseline. D. The changed bile acid compositions and their signal maps between two enterotypes after Acarbose treatment. The black represents a significant decrease after treatment, white represents a significant increase after treatment, and gray represents no significant change after treatment, P <0.05.

### Detailed Descriptions of the Preferred Embodiments

The present invention will be described in detail with reference to the following embodiments.

### Embodiment

For naive patients with Type 2 diabetes, their liver and kidney functions, blood glucose and lipid levels, intestinal hormones, inflammatory factors, and cardiovascular risk-related factors are evaluated before medication. Their stool, urine, and blood samples are retained. After clear diagnosis and evaluation, patients are treated for 3 months at a daily dose of Acarbose 300mg. The patients' blood glucose levels are followed up every month within 3 months, and the medication is adjusted according to the blood glucose level. Three months later, the pre- medication assessment is repeated, and the urine, stool and blood samples are retained.

Specific steps are as follows:
1. Collection of clinical samples
   a) A randomized, opened, positive control method is adopted to collect the naïve patients with Type 2 diabetes and normal controls of their spouses. The clinical and biochemical data, gastrointestinal motility of diabetic patients before and after Acarbose treatment are compared and their blood and stool samples are collected. The newly diagnosed patients of Type 2 diabetes without medication receive routine examinations, including the retention of stool and blood samples.
      Collection of stool samples
   b) Stool
      i. The instrument: Plastic basin (the diameter less than the caliber of household flush toilet)
      ii. Freshness protection package, sterile small-handle spoon
      iii. 50 ml sterile centrifuge tube
      iv. Place the plastic basin (the diameter less than the caliber of household flush toilet) into a flush toilet, cover the freshness protection package (do not immerse the edges of the freshness protection package into the water of the toilet). If samples are taken in the hospital, cover a freshness protection package directly in the clean potty, to retain the stool samples;
      v. Mix the upper layer of the fresh stool sample well with a small-handle spoon, pick up a small amount into a 50 ml sterile centrifuge tube. Take at least 10 g sample each tube, tighten the tube cover (indicate the sampling time, sampling group and number on the centrifuge tube wall). Retain stools for each subject each time, a total of 3 tubes of samples (RNAlater treatment tube, glycerine tube, and treatment-free tube).
      vi. Immediately place the collected samples into -80°C for cryopreservation.
   c) Retention of serum samples
      i. Draw venous blood 15 ml under fasting condition, of which, 1.5 ml is used for detection of plasma glucose, 6.5 ml is added to ordinary tubes (including aprotinin, DPPV inhibitor), and 6.5 ml is added to anticoagulant tubes (including heparin and RNA later)
      ii. Centrifuge the blood in ordinary tube at 4°C, when serum is separated out, draw about 3 ml, and divide to three 1.5 ml Eppendorf on average, then tighten the tube caps;
      iii. Centrifuge the anticoagulation blood immediately at 4°C, to separate out about 3 ml of plasma, then divide to three 1.5 ml imported RNAase free Eppendorf tubes on average,
      iv. Indicate the sample name, center number and random number on the tube in details;
      v. Cover the tubes for one week with plastic tapes;
      vi. Keep them at -20 °C (placed at -80 °C if condition permitted), and subpackage the plasma and immediately place them at -20°C or dry ice.

### 2. Determination of bile acid

The reagents Sodium taurochenodeoxycholate(TCDCA), Sodium glycocholate hydrate(GCA), Sodium taurodeoxycholate(TDCA), Chenodeoxycholic acid(CDCA), Ursodeoxycholic acid(UDCA), Taurocholic acid (TCA), Sodium glycodeoxycholate(GDCA), Glycoursodeoxycholic acid(GUDCA), Cholic acid(CA), Deoxycholic acid(DCA), Sodium glycochenodeoxycholate(GCDCA), Sodium tauroursodeoxycholate(TUDCA), Sodium taurolithocholate(TLCA), Lithocholic acid(LCA), NH4HCO3 are purchased from Sigma, USA; Glycochenodeoxycholic Acid 3-Sulfate Disodium Salt(GCDCS) are synthesized in the laboratory of Zhejiang University; Chenodeoxycholic Acid-d4 (CDCA-d4), Glycochenodeoxycholic Acid-d5 3-Sulfate Disodium Salt(GCDCS-d5), Taurochenodeoxycholic Acid-d5 (TCDCA-d5), Cholic Acid-d5(CA-d5), Glycocholic acid-d5(GCA-d5) , Lithocholylglycine(GLCA) are purchased from TRC, Canada; and Taurodeoxycholic Acid-d5(TDCA-d5), Taurocholic acid - d5(TCA-d5) are purchased from CIL, USA. Acetonitrile and methanol are purchased from Merk, Germany.

Sample pretreatment: Take 75 µL of blood sample, add 300 µL of internal standard methanol, to extract the target compound and precipitate the protein, vortex 30s, centrifuge 10 min at the rate of 15000 rpm, draw 200 µL of the supernatant, then lyophilize, re-dissolve in 50 µL of 25% acetonitrile solution, and wait for sample injection. Instrument and method: conduct sample analysis using 1290 Infinity liquid phase (Agilent, USA) and 6460A triple quadrupole mass spectrometry system (Agilent, USA). Perform the liquid phase separation using 100mm×2.1mm ACQUITY UPLC C8 column having a particle size of 1.7µm (Waters, USA), of which, phase A is 10 mM NH4HCO3 aqueous solution, phase B is pure acetonitrile; initially 25% phase B, retaining 0.5 min, followed by increased to 40% phase B linearly within 12.5 min, then increased to 90% within 1 min, flush the system for 3min, recover to 25% phase B in 0.5 min, after equilibrating 2.5 min, the flow rate is 0.35 ml/min, column temperature is 35°C and the injection volume is 5 µL; Mass spectrometry is performed by ESI source negative ion mode, with main parameters as follows: Gas Temp: 350°C; Gas Flow: 8 I/min; Nebulizer: 40 psi; Sheath Gas Temp: 400 °C; Sheath Gas Flow: 8 I/min; Capillary: 3500 V; Nozzle voltage: 400 V. The bile acid is detected under a reaction monitoring mode (MRM). The concentration of the internal standard and the main mass spectrum parameters are shown in Table 1. The setting of mass spectrum parameters for bile acid analysis is shown in Table 2.

**Table 1 Concentration of internal standard and main MS parameters**

| | Concentration (mg/L) | parent ion | daughter ion | Fragment or | Collision Energy (eV) |
|---|---|---|---|---|---|
| CA-d5 | 0.08 | 412.5 | 412.5 | 200 | 10 |
| CDCA-d4 | 0.3 | 395.4 | 395.4 | 200 | 10 |
| GCA-d5 | 0.2 | 469.2 | 74.1 | 200 | 35 |
| GCDCA- | 0.2 | 452.3 | 74.1 | 240 | 40 |
| GCDCS- | 0.2 | 533.3 | 453.3 | 200 | 35 |
| TCA-d5 | 0.1 | 519.2 | 80.1 | 320 | 90 |
| TCDCA- | 0.1 | 503.3 | 80.2 | 300 | 70 |
| TDCA-d5 | 0.1 | 503.3 | 80.2 | 300 | 70 |

**Table 2 MS parameters for bile acid analysis and internal standard for calibration**

| | Compound | Internal standard | parent ion | daughter ion | Fragment or | Collision Energy eV |
|---|---|---|---|---|---|---|
| Lithocholic acid | LCA | CA-d5 | 375.3 | 375.3 | 200 | 2 |
| Chenodesoxycholic acid | CDCA | CDCA-d4 | 391.4 | 391.4 | 200 | 10 |
| Deoxycholic acid | DCA | CDCA-d4 | 391.4 | 391.4 | 200 | 10 |
| Ursodeoxycholic acid | UDCA | CDCA-d4 | 391.4 | 391.4 | 200 | 5 |
| Bile acid | CA | CA-d5 | 407.5 | 407.5 | 200 | 10 |
| Glycine conjugated with lithocholic acid | GLCA | GCA-d5 | 432.3 | 74 | 200 | 35 |
| Glycine conjugated with deoxycholic acid | GDCA | GCDCA-d4 | 448.2 | 74.1 | 200 | 40 |
| Glycine conjugated with chenodesoxycholic acid | GCDCA | GCDCA-d4 | 448.3 | 74.1 | 240 | 42 |
| Glycine conjugated with ursodeoxycholic acid | GUDCA | GCDCA-d4 | 448.3 | 74.1 | 200 | 40 |
| Glycine conjugated with bile acid | GCA | GCA-d5 | 464.2 | 74.1 | 200 | 35 |
| Taurine conjugated with lithocholic acid | TLCA | TCA-d5 | 482.1 | 80.1 | 300 | 70 |
| Taurine conjugated with chenodesoxycholic acid | TCDCA | TCDCA-d5 | 498.3 | 80.2 | 300 | 70 |
| Taurine conjugated with lithocholic acid | TDCA | TDCA-d5 | 498.3 | 80.2 | 300 | 70 |
| Taurine conjugated with ursodeoxycholic acid | TUDCA | TCA-d5 | 498.3 | 80.1 | 320 | 70 |
| Taurine conjugated with bile acid | TCA | TCA-d5 | 514.2 | 80.1 | 320 | 90 |

### 3. DNA sequencing of gut microbiota

For HiSeq 2500 sequencing, the fragments at the length of 350bp are used to establish the database and compare with 9.9M human intestinal gene set, to obtain the phylum, species and genus of IMG (70% coverage rate and 65% recognition rate at the phylum level, 85% recognition rate at the genus level, and 95% recognition rate at the species level). The clustering analysis of gut microbiota is performed by principal component analysis (PCA).

In the present invention, the gut microbiota colony DNA extraction and second generation sequencing of metagenome are performed in patients' feces, then compared with the published 9.9M human gut metagenome gene set, with a matching rate about 77%. About 143 kinds of gut microbiota with annotation information and difference before and after medication are found by clustering analysis. The genus level analysis shows that, different clustering of gut microbiota is found at the baseline in patients with Type 2 diabetes. According to the characteristic accumulation of gut microbiota, the enterotype is obtained. In the present invention, there are mainly two enterotypes: one is the Prevotella-based Prevotella enterotype, and the other is Bacteroides-based Bacteroides enterotype (FIG. 1).

According to enterotype classification, there are no significant differences in the sex and age distribution between the two types of patients with Type 2 diabetes. There are no significant differences in the baseline blood glucose levels, body weights, liver and kidney functions and other health indicators between them. Only the levels of red blood cells, hemoglobin and interleukin 6 are slightly higher in the patients of Prevotella enterotype (P <0.05). (Table 3)

After treatment, the efficacy of this drug for treatment of diabetes in the two groups of patients at baseline is observed.

First, the most major efficacy of Acarbose is reflected by the reduction in 2h postprandial plasma glucose and HbAlc. But there are no differences in the two indexes between the two enterotypes (FIG. 2, Table 4).

Second, there is difference in control of fasting blood glucose level between the two enterotypes. The patients of Bacteroides enterotype show significant improvement and the degree of improvement after treatment; while the patients with Type 2 diabetes of the Prevotella enterotype do not show significant improvement in fasting blood glucose level. The other metabolic related indexes, including insulin, body weight, BMI, waist circumference, cardiovascular risk factors and intestinal hormones are significantly different between the two enterotypes.

The fasting C peptide and insulin levels are significantly decreased after treatment in the Bacteroides enterotype. After calculation, the HOMAIR index, which reflects insulin resistance, decreases after Acarbose treatment, but this benefit is significant only in the patients of Bacteroides enterotype, but not significant in patients of Prevotella enterotype, suggesting that patients with Type 2 diabetes of Bacteroides enterotype are more likely to improve their status of insulin resistance after taking Acarbose. The standard meal-induced insulin release curve, waist-to-hip ratio, and Adiponectin levels that are related to the insulin resistance show significant decrease after Acarbose treatment in T2DM patients of Bacteroides enterotype, but these indexes show no significant change in the patients of Prevotella enterotype.

Acarbose can cause a decrease in TG, APOA and DBP, and there are no significant differences between the two enterotypes after treatment; however, some plasma factors associated with diabetic vascular complications such as PDGFAA and PDGFAABB, endothelin, VegfC are significantly lower in the Bacteroides enterotype, suggesting that the Acarbose treatment can bring more benefits of reducing vascular complications in addition to lowering blood glucose level and risks of macrovascular diseases in the Bacteroides enterotype.

Gut hormone is a hot research target in the treatment of Type 2 diabetes, and its level is changed significantly in the Acarbose treatment. Among the several gut hormones detected, the elevated GLP1, glucagon, PYY, and ghrelin and GIP at each time point after medication are significant in the Bacteroides enterotype, but not significant in the Prevotella enterotype, suggesting that any metabolic benefit of Acarbose through gut hormones is more significant in the Bacteroides enterotype.

Third, there is a difference in bile acid spectrum between the two enterotypes at the baseline level (FIG. 3A, B). In the Bacteroides enterotype, the levels of deoxycholic acid and lithocholic acid in secondary bile acid are significantly lower than those in Prevotella enterotype, whereas the ursodeoxycholic acid level with protective effect is higher than that in Prevotella enterotype. The further gut metagenome analysis showed that, the ursodesoxycholic acid is further degraded into lithocholic acid KO, which is enriched in Prevotella enterotype apparently (FIG. 3C), suggesting that there is significant difference in the bile acid metabolic ability of gut microbiota between patients of two enterotypes. After treatment with Acarbose, the difference in bile acid composition is more pronounced between both enterotypes (FIG. 3D). The two kinds of primary bile acids are significantly increased in two enterotypes after treatment with Acarbose, but without enterotype-specific changes, suggesting that Acarbose may affect the whole reabsorption of bile acid in the small intestine.

Therefore, this study shows that, different enterotypes can predict patients' benefits from Acarbose treatment of diabetes, especially improving the insulin resistance, reducing the secondary bile acid, and promoting the reduction of cardiovascular risks in addition to glucose-lowering. The enterotype diagnosis can be completed by ordinary DNA PCR amplification of 16sRNA of characteristic bacteria genus, which is convenient and economical, making the precision medical care of Type 2 diabetes possible.

**Table 3 Baseline clinical indexes of two enterotypes**

| **Clinical Index** | **P value** | **Bacteroides enterotype** | **Prevotella enterotype** |
|---|---|---|---|
| SBP | 0.052305571 | 126.68±15.68 | 134.92±19.75 |
| DBP | 0.569158065 | 80.87±8.49 | 82.54±11.12 |
| Height | 0.406982243 | 166.76±7.27 | 168.13±7.97 |
| Body weight | 0.632776326 | 72.76±9.93 | 74.44±12.11 |
| BMI | 0.426864667 | 26.17±3.48 | 26.2±2.73 |
| Waist circumference | 0.382242756 | 90.58±7.7 | 92.53±10.05 |
| Hip circumference | 0.850454946 | 99.15±7.58 | 98.71±6.25 |
| Waist-to-hip ratio | 0.073022015 | 0.91±0.05 | 0.94±0.06 |
| RBC count | **0.008822773*** | 4.77±0.45 | 5.01±0.3 |
| Hemoglobin | **0.015559629*** | 142.47±20.66 | 150.75±11.96 |
| Hematocrit | 0.110126957 | 9.5±22.96 | 9.69±22.65 |
| WBC count | 0.221434929 | 6.35±1.46 | 6.67±1.35 |
| Granulocyte percentage | 0.721728164 | 57.18±12.59 | 54.07±17.36 |
| Percentage of lymphocytes | 0.823390429 | 31.2±10.11 | 30.08±12.05 |
| Percentage of monocytes | 0.92702484 | 5.68±2.12 | 5.64±2.2 |
| Platelet count | 0.977269042 | 207.14±53.01 | 208.26±53.28 |
| ALT | 0.770933665 | 34.74±20.18 | 39.33±38.6 |
| AST | 0.821446414 | 26.76±12.39 | 32.45±33.84 |
| Alkaline phosphatase | **0.003733751*** | 68.02±17.83 | 81.7±21.67 |
| Glutamyl transpeptidase | 0.219732988 | 38.87±37.46 | 48.13±54.35 |
| Total bilirubin | 0.517075592 | 15.66±5.85 | 14.33±4.54 |
| Direct bilirubin | 0.930882347 | 3.17±1.58 | 3.3±1.57 |
| Total protein | 0.555631501 | 72.16±4.18 | 72.99±4.34 |
| Albumin | 0.439028944 | 46.81±26.74 | 44.38±4.21 |
| Uric acid | 0.389295382 | 5.01±1.2 | 5.24±1.03 |
| Creatinine | 0.424300196 | 66.58±14.76 | 69.16±12.41 |
| Urea nitrogen | 0.93814784 | 301.17±73.87 | 301.29±65.47 |
| Potassium | 0.802728704 | 6.46±17.43 | 4.11±0.51 |
| Sodium | 0.153693762 | 137.39±17.91 | 140.64±3.13 |
| Chloride | 0.45778794 | 102.73±2.78 | 102.32±2.5 |
| Triglycerides | 0.479383839 | 2.29±1.56 | 2.28±2.29 |
| Total cholesterol | 0.763815537 | 5.03±0.91 | 5.05±1.54 |
| High density lipoprotein | 0.974185078 | 3.05±0.81 | 3.17±1.05 |
| Low density lipoprotein | 0.341454604 | 1.2±0.45 | 1.18±0.2 |
| Apolipoprotein A | 0.99402696 | 1.34±0.18 | 1.36±0.17 |
| Apolipoprotein B | 0.923810467 | 1.03±0.21 | 1.06±0.29 |
| Lipoprotein A | 0.833966588 | 62.95±144.85 | 164.75±516.3 |
| Blood glucose 0 min | 0.091734172 | 7.91±1.4 | 7.29±1.11 |
| Blood glucose 30 min | 0.130269742 | 10.91±1.96 | 10.27±1.87 |
| Blood glucose 60 min | 0.257347624 | 14.24±2.43 | 13.79±1.84 |
| Blood glucose 120 min | 0.243421632 | 14.78±2.86 | 14±2.36 |
| Blood glucose 180 min | 0.741659488 | 12.11±3.21 | 11.51±3.04 |
| Serum insulin 0 min | 0.509699318 | 9.54±4.74 | 11.83±11.34 |
| Serum insulin 30 min | 0.234997693 | 19.7±11.99 | 22.7±12.31 |
| Serum insulin 60 min | 0.469868561 | 36.99±21.41 | 42.04±27.34 |
| Serum insulin 120 min | 0.517877918 | 48.41±26.27 | 55.15±35.47 |
| Serum insulin 180 min | 0.889080689 | 38.78±26.07 | 35.42±22.9 |
| Serum C-peptide 0 min | 0.979767063 | 2.5±0.74 | 2.67±1.27 |
| Serum C-peptide 30 min | 0.318552538 | 3.42±1.1 | 3.6±1.17 |
| Serum C-peptide 60 min | 0.694278947 | 5.08±1.77 | 5.24±1.86 |
| Serum C-peptide 120 min | 0.809629053 | 7.59±2.29 | 7.62±2.45 |
| Serum C-peptide 180 min | 0.343793846 | 7.3±2.51 | 6.61±1.97 |
| Plasma insulin 0 min | 0.469445501 | 478.88±234.65 | 476.64±338.58 |
| Plasma insulin 30 min | 0.810557675 | 791.99±393.84 | 808.26±506.26 |
| Plasma insulin 60 min | 0.838524751 | 1396.45±752.85 | 1467.09±1061.37 |
| Plasma insulin 120 min | 0.741764202 | 1934.72±997.05 | 2110.35±1443.51 |
| Plasma insulin 180 min | 0.754907581 | 1641.1±947.84 | 1639.39±1040.15 |
| Plasma C-peptide 0 min | 0.384853423 | 1175.79±418.14 | 1127.3±498.93 |
| Plasma C-peptide 30 min | 0.813883421 | 1670.96±718.73 | 1669.2±711.29 |
| Plasma C-peptide 60 min | 0.8047981 | 2578.14±1156.59 | 2530±1212.03 |
| Plasma C-peptide 120 min | 0.852096376 | 3871.17±1525.71 | 3800.54±1372 |
| Plasma C-peptide 180 min | 0.587827408 | 3626.21±1473.21 | 3336.12±1109.94 |
| HbAlc | 0.394975993 | 7.65±0.91 | 7.45±0.72 |
| GHRP 0 min | 0.647538837 | 47.52±28.77 | 54.93±51.33 |
| GHRP 30 min | 0.270618653 | 54.38±33.75 | 51.8±43.65 |
| GHRP 60 min | 0.100675425 | 45.72±25.99 | 39.13±30.64 |
| GHRP 120 min | 0.420950166 | 35.62±17.43 | 34.83±24.87 |
| GHRP 180 min | 0.608065141 | 42.79±21.75 | 47.11±36.95 |
| GLP-1 0 min | 0.925944614 | 6.46±10.84 | 5.02±5.17 |
| GLP-1 30 min | 0.874727025 | 19.49±18.36 | 18.24±15.26 |
| GLP-1 60 min | 0.891679993 | 16.79±13.74 | 14.11±8.46 |
| GLP-1 120 min | 0.706003564 | 11.35±12.71 | 8.58±7.07 |
| GLP-1 180 min | 0.412865834 | 9.46±12.94 | 7.22±6.79 |
| Glu 0 min | 0.976095762 | 29.11±13.28 | 30.48±17.95 |
| Glu 30 min | 0.738477919 | 37.97±20.1 | 37.5±23.81 |
| Glu 60 min | 0.605878342 | 35.31±19.34 | 34.12±23.53 |
| Glu 120 min | 0.343451344 | 25.45±12.45 | 24.88±16.59 |
| Glu 180 min | 0.911837296 | 22.34±12.16 | 23.56±14.08 |
| PYY 0 min | 0.902682115 | 53.23±56.11 | 41.27:t30.44 |
| PYY 30 min | 0.826513742 | 67.21±59.48 | 61.55±43.07 |
| PYY 60 min | 0.984419278 | 64.4±55.36 | 55.23±32.99 |
| PYY 120 min | 0.556885888 | 55.41±50.64 | 51.09±30.71 |
| PYY 180 min | 0.705995014 | 57.51±56.59 | 43.62±32.17 |
| GIP 0 min | 0.466825554 | 76.52±47.45 | 72.4±53.13 |
| GIP 30 min | 0.959035633 | 358.7±222.63 | 357.55±244.17 |
| GIP 60 min | 0.693172872 | 443.67±220.91 | 417.88±238.84 |
| GIP 120 min | 0.453200572 | 406.03±192.67 | 388.55±218.63 |
| GIP 180 min | 0.181743927 | 323.22±179.85 | 289.97±235.54 |
| PP 0 min | 0.318610459 | 93.28±78.66 | 82.39±78.19 |
| PP 30 min | 0.569166173 | 313.48±220.69 | 258.05±114.37 |
| PP 60 min | 0.133570837 | 284.41±196.22 | 217.63±115.08 |
| PP 120 min | 0.245241771 | 222.38±163.09 | 180.86±108.39 |
| PP 180 min | 0.106766755 | 180.24±111.6 | 143.88±97.4 |
| IL 17M | 0.962918479 | 138.85±74.04 | 155.04±123.32 |
| G-CSF | 0.803059249 | 50.43±21.81 | 49.25±17.99 |
| IFN r1 | 0.181566671 | 45.55±42.56 | 34.79±17.12 |
| IL 10 | 0.861536003 | 1.68±1.51 | 1.69±1.65 |
| MIP-3a | 0.333709066 | 22.75±19.47 | 25.06±16.77 |
| IL 12 | 0.993168795 | 8.76±7.01 | 8.42±5.75 |
| IL 15 | 0.267007222 | 232.97±132.57 | 258.05±119.82 |
| IL 17A | 0.7415298 | 32.16±9.43 | 31.08±9.36 |
| IL 22 | 0.13451041 | 42.34±40.57 | 30.96±10.48 |
| IL 9 | 0.480277271 | 81.35±54.9 | 69.56±35.58 |
| IL 33 | 0.751136369 | 37.46±13.13 | 35.71±13.65 |
| IL 2 | 0.472384797 | 58.54±49.48 | 47.94±29.24 |
| IL 21 | 0.905751857 | 28.21±11.92 | 31.75±22.21 |
| IL 4 | 0.927445079 | 20.29±15.95 | 19.9±13.78 |
| IL 23 | 0.902434691 | 37.04±23.49 | 36.37±20.38 |
| IL 5 | 0.892399481 | 45.18±36.22 | 38.48±19.04 |
| IL IL17E | 0.608790357 | 25.15±31.08 | 19.56±10.22 |
| IL 27 | 0.534356146 | 71.75±56.63 | 60.5±37.44 |
| IL 31 | 0.793285936 | 112.57±57.24 | 101.87±34.87 |
| TNF B | 0.885718446 | 45.25±32.83 | 40.52±18.34 |
| IL 28a | 0.591202712 | 20.21±9.34 | 19.56±8.97 |
| FGF 19M | 0.802617256 | 23.01±4.13 | 23.27±3.78 |
| FGF 23M | 0.132019218 | 45.88±9.26 | 41.96±6.75 |
| Oncostatin | 0.496200994 | 105.9±75.52 | 93.87±65.35 |
| cTn | 0.139027346 | 19.77±18.76 | 14.9±10.08 |
| ET | 0.560717515 | 12.79±2.7 | 12.88±3.41 |
| FGF 21 | 0.738341043 | 0.3±0.32 | 0.26±0.26 |
| NGF | 0.100733692 | 2.41±3.11 | 2.83±2.61 |
| HGF | 0.271836439 | 408.2±258.41 | 465.78±249 |
| MCP 1 | 0.454428023 | 244.24±136.45 | 246.4±77.85 |
| TNF a | 0.292633798 | 4.56±2.19 | 5.67±4.46 |
| SICAM | 0.684945545 | 161.33±75.77 | 188.08±166.69 |
| MPO | 0.27368969 | 385.65±351.89 | 464.48±363.39 |
| sP-selectin | 0.165666585 | 141.01±76.01 | 270.22±572.86 |
| sVCAM | 0.233334305 | 703.18±244.75 | 820.42±410.18 |
| PDGF AA | 0.945399512 | 4951.68±1904.72 | 4902.38±1831.41 |
| PDGF AABB | 0.490732791 | 26581.76±14551.83 | 27347.81±11562.13 |
| RANTES | 0.899107284 | 54893.62±24860.48 | 52830.46±19005.15 |
| LEP | 0.632956992 | 5307140.26±30525 | 6121581.85±42971 |
| | | 69.78 | 13.91 |
| NGAL | 0.43181483 | 136543.91±75938.8 4 | 158878.69±102095. 95 |
| Resistin | 0.051869812 | 16813.35±9816.98 | 21730.58±11698.65 |
| Adipokine | 0.377073702 | 3322727.99±15540 37.78 | 3526181.62±16305 15.78 |
| PAI 1 | 0.635961359 | 57833.47±21242.95 | 59240±19939.5 |
| CRP | 0.310428088 | 11.92±15.92 | 21.22±49.48 |
| FETU-A | 0.790035938 | 333.82±50.16 | 332.62±40.54 |
| L- selectin | 0.973025194 | 1.54±0.25 | 1.58±0.32 |
| FABP 3 | 0.706810959 | 2282.3±1074.01 | 2481.19±1192.16 |
| FABP 4 | 0.706016525 | 14084.38±12064.5 | 13559.88±12861.95 |
| ECGF | 0.1033731 | 187.05±127.4 | 234.75±131.23 |
| ET | 0.640239461 | 3.78±1.99 | 4.08±3.37 |
| FGF 1 | 0.400574693 | 13.71±10.83 | 12.86±5.24 |
| VEGF c | 0.614322703 | 113.88±37.89 | 113.02±46.82 |
| VEGF d | 0.115360982 | 163.15±117.25 | 203.19±123.58 |
| FGF 2 | 0.685128912 | 37.29±22.25 | 41.01±26.94 |
| VEGF a | 0.416512674 | 499.98±398.81 | 400.25±267.47 |
| LEP | 0.732125461 | 6490.77±6637.01 | 6502.46±5785.56 |
| IL 8 | 0.812673509 | 17.03±26.21 | 18.69±24.86 |
| IL 1b | 0.912521353 | 0.48±0.79 | 0.59±1.29 |
| IL 13 | 0.442815325 | 237.23±132.1 | 254.36±129.07 |
| IL 6 | **0.044391053*** | 2.85±5.27 | 3.1±3.63 |
| LBP | 0.185062045 | 20.61±7.28 | 18.66±7.99 |
| HOMA-IR | 0.895763831 | 3.33±1.73 | 3.83±3.38 |
| Plasma HOMA-IR | 0.206702804 | 3.76±2.07 | 3.44±2.46 |

**Table 4**

| | P value | | Mean | | | |
|---|---|---|---|---|---|---|
| | Bacterioide enterotype | Prevotella enterotype | Acarbose | | | |
| | Acarbose | Acarbose | Bacterioide enterotype | | Prevotella enterotype | |
| | | | Baseline | After treatment | Baseline | After treatment |
| Adipisin | 0.735655998 | 0.678771973 | 3168874 .417 | 3174901. 441 | 3105379 .8 | 3221619. 467 |
| **Adpn*** | **0.009070051** | 0.072998047 | 4659142 .417 | 5157339. 824 | 6825515 .733 | 10063264 .07 |
| **AKP*** | **0.000298293** | 0.148678549 | 68.3823 5294 | 59.22857 143 | 85.4266 6667 | 77.5 |
| ALB | 0.052938425 | 0.125506471 | 49.7454 5455 | 42.26 | 45.5857 1429 | 44.38571 429 |
| ALT | **0.023923875** | 0.315055201 | 37.68 | 29.42857 143 | 40.7 | 44.35714 286 |
| **APOA*** | **0.011314861** | 0.125 | 1.32821 4286 | 1.2475 | 1.37714 2857 | 1.267272 727 |
| APOB | 0.42397348 | 0.1875 | 1.02964 2857 | 0.963571 429 | 1.13142 8571 | 0.927272 727 |
| **AST*** | **0.009259021** | 0.183967151 | 28.2029 4118 | 22.45714 286 | 35.9133 3333 | 29.35714 286 |
| **BMI*** | **3.26E-05** | **0.003494192** | 26.4255 5556 | 25.51138 889 | 26.0686 6667 | 25.11333 333 |
| bun | **0.017056015** | 0.221189047 | 309.282 3529 | 329.2 | 306.212 | 326.4615 385 |
| **BW*** | **2.32E-05*** | **0.00367322*** | 74.4527 7778 | 71.91111 111 | 75.38 | 72.63333 333 |
| cl | 0.365775393 | 0.149551339 | 102.937 5 | 103.4 | 101.692 3077 | 103.3571 429 |
| **CPO*** | **0.000751479** | 0.229309082 | 2.62083 3333 | 2.216388 889 | 2.66866 6667 | 2.485333 333 |
| **CP120*** | **5.92E-08** | **0.001159668** | 7.84277 7778 | 5.204166 667 | 7.61533 3333 | 5.304666 667 |
| **CP180*** | **1.25E-09** | **0.012036948** | 7.32777 7778 | 4.843333 333 | 6.26266 6667 | 4.734666 667 |
| **CP30*** | **3.59E-05** | **0.023068064** | 3.60222 2222 | 2.7525 | 3.502 | 2.802 |
| **CP60*** | **5.42E-06** | **6.10E-05** | 5.375 | 3.71 | 5.294 | 3.609333 333 |
| creatine | 0.530974718 | 1 | 67.1088 2353 | 67.61428 571 | 67.9866 6667 | 67.41538 462 |
| crp | 0.852740904 | 0.229309082 | 13.5725 | 29.96117 647 | 10.216 | 19.638 |
| DBIL | 0.42621214 | 0.833885439 | 2.75588 2353 | 2.965714 286 | 3.54285 7143 | 6.292857 143 |
| **DBP*** | **0.000195001** | **0.049089037** | 82.0833 3333 | 75.37142 857 | 80.4666 6667 | 74.93333 333 |
| egf | 0.458006509 | 0.267578125 | 193.700 5882 | 183.3530 303 | 206.374 2857 | 267.604 |
| ENDOM | **0.030053592** | 0.207824804 | 12.3611 1111 | 13.43939 394 | 13.7333 3333 | 14.26666 667 |
| **Endothe lin*** | **0.039909487** | 0.363606971 | 3.46 | 4.158571 429 | 4.92642 8571 | 4.776 |
| FABP3 | 0.903465115 | 0.890380859 | 2374.76 0833 | 2350.673 429 | 2293.33 3333 | 2146.933 333 |
| FABP4 | 0.103153911 | 0.252380371 | 14146.3 1429 | 11504.54 286 | 12673.3 3333 | 10510.4 |
| **fetuinA∗** | **0.000150225** | **0.000610352** | 340.418 0556 | 400.9188 235 | 334.999 3333 | 417.3386 667 |
| fgf1 | 0.327136563 | 0.777511253 | 11.0894 4444 | 11.65771 429 | 14.0064 2857 | 13.78933 333 |
| FGF19M | **0.000721153** | **0.006268599** | 22.4444 4444 | 19.73529 412 | 22.6 | 19.3 |
| FGF1M | 0.327494344 | 0.62856768 | 21.9444 4444 | 22.57352 941 | 24.4 | 24.33333 333 |
| fgf2 | 0.271079158 | 0.94425068 | 33.1302 7778 | 35.39542 857 | 42.5535 7143 | 43.98266 667 |
| fgf21 | 0.502579383 | 0.779828433 | 0.31777 7778 | 0.37 | 0.33928 5714 | 0.296 |
| fgf21M | **0.000200362** | **0.002624512** | 143.041 6667 | 100.0588 235 | 136.4 | 97.93333 333 |
| FGF23M | **5.55E-05** | 0.628914718 | 44.9027 7778 | 37.07352 941 | 42.3666 6667 | 43.46666 667 |
| FGF2M | 0.219893155 | 0.805893176 | 12.3055 5556 | 12.69117 647 | 13.1333 3333 | 13.66666 667 |
| **G120*** | **1.75E-07** | **6.10E-05** | 14.6427 7778 | 9.171388 889 | 13.292 | 9.170666 667 |
| **G180*** | **7.36E-09** | **0.000244141** | 11.6838 8889 | 7.941388 889 | 10.65 | 7.852857 143 |
| **G30*** | **4.07E-10** | **0.00012207** | 10.7305 5556 | 7.622777 778 | 9.652 | 7.601428 571 |
| **G60*** | **5.82E-11** | **0.00012207** | 13.9961 1111 | 9.074166 667 | 13.1206 6667 | 9.055 |
| GIPO | 0.890667984 | 0.71484375 | 79.7154 2857 | 80.26388 889 | 84.0453 3333 | 57.39785 714 |
| **GIP120*** | **0.007692964** | 0.067626953 | 382.071 1765 | 264.0158 333 | 354.922 | 227.7985 714 |
| **GIP180*** | **0.002999473** | 0.067626953 | 290.193 8235 | 201.5647 222 | 259.104 | 194.8235 714 |
| **GIP30*** | **0.01013191** | **0.020263672** | 336.143 4286 | 219.1305 556 | 342.732 | 208.4314 286 |
| **GIP60*** | **0.000305745** | **0.00402832** | 439.173 1429 | 258.945 | 382.323 3333 | 217.2485 714 |
| GMCSF M | 0.400942689 | 0.798033799 | 49.375 | 64.61764 706 | 55.3 | 67.96666 667 |
| **GO*** | **2.34E-06** | 0.30279541 | 7.9075 | 6.606944 444 | 7.00266 6667 | 6.712 |
| HB | 0.866145989 | 0.063711823 | 144.971 4286 | 140.6431 429 | 152.333 3333 | 147.8333 333 |
| **HBA1C*** | **3.54E-07** | **0.001087736** | 7.625 | 6.425 | 7.30666 6667 | 6.313333 333 |
| HDL | 0.888463907 | 0.109863281 | 2.97 | 2.989411 765 | 3.30461 5385 | 2.868571 429 |
| HGF | 0.162910502 | 0.561401367 | 415.155 2778 | 434.1691 176 | 377.43 | 400.8593 333 |
| HIP | 0.135245032 | 0.9372558 | 100.202 8571 | 98.82777 778 | 98.0666 6667 | 97.21428 571 |
| HOMA.I R | **6.62E-05** | 0.638671875 | 3.58523 6667 | 2.487479 506 | 3.79496 563 | 3.326864 889 |
| IFNr1 | 0.259007015 | 0.609162891 | 38.7638 8889 | 42.10294 118 | 37.6 | 38.8 |
| IL10 | 0.241252899 | 0.635498047 | 1.52538 4615 | 2.300869 565 | 1.78785 7143 | 1.797692 308 |
| IL12 | 0.444698256 | 0.524475098 | 7.88114 2857 | 10.11903 226 | 9.13866 6667 | 9.944 |
| IL13 | 0.394072711 | 0.735351563 | 231.221 4286 | 311.6553 846 | 251.214 6667 | 279.3569 231 |
| IL15M | 0.098387918 | 0.488708496 | 224.303 4286 | 333.3187 5 | 269.899 3333 | 291.8106 667 |
| IL17AM | 0.823963715 | 0.726607536 | 32.8611 1111 | 33.73529 412 | 33.2 | 32.03333 333 |
| IL17EM | 0.870855906 | 0.949882816 | 21.5138 8889 | 22.23529 412 | 21.3666 6667 | 21.23333 333 |
| IL17FM | 0.055283514 | 0.30279541 | 124.958 3333 | 181.3529 412 | 183.133 3333 | 232.6333 333 |
| IL1B | 0.694494109 | 1 | 0.37472 2222 | 0.532352 941 | 0.32428 5714 | 0.910666 667 |
| IL1bM | 0.359000687 | 0.977243513 | 13.7777 7778 | 14.66176 471 | 12.5333 3333 | 22.23333 333 |
| IL21 | 0.850725867 | 0.394055106 | 26.0416 6667 | 29.13235 294 | 35.9 | 34.46666 667 |
| IL22M | 0.175108876 | 0.191177717 | 36.4722 2222 | 42.5 | 32.8333 3333 | 30.5 |
| IL23M | 0.085674334 | 0.460211098 | 34.8055 5556 | 39.35294 118 | 39.5666 6667 | 41.7 |
| IL27 | 0.81743512 | 0.348590881 | 59.4305 5556 | 61.27941 176 | 69.3666 6667 | 73.1 |
| IL28a | 0.80929836 | 0.18707508 | 20.0277 7778 | 20.04411 765 | 20.9 | 18.36666 667 |
| IL2M | 0.567407854 | 0.267970259 | 45.1944 4444 | 53.45588 235 | 53.2 | 58.76666 667 |
| IL31M | 0.270120255 | 0.719726563 | 98.8611 1111 | 112.9558 824 | 105.733 3333 | 112.3333 333 |
| IL33M | 0.304945974 | 0.181618578 | 36.1805 5556 | 42.13235 294 | 38.7 | 43.5 |
| IL4M | 0.104184122 | 0.120544434 | 17.6905 7143 | 30.73387 097 | 22.642 | 31.074 |
| IL5M | 0.644309509 | 0.890380859 | 34.5694 4444 | 38.44117 647 | 42.3333 3333 | 43.96666 667 |
| IL6 | 0.346414942 | 0.12890625 | 2.57758 6207 | 4.845555 556 | 1.63111 1111 | 2.665 |
| IL6M | 0.724177037 | **0.04439591** | 42.0833 3333 | 55.85294 118 | 31.0666 6667 | 40.53333 333 |
| IL8 | 0.648417992 | 0.463134766 | 15.0672 2222 | 11.92176 471 | 9.64071 4286 | 15.43866 667 |
| IL9M | 0.644339761 | 0.488708496 | 67.2361 1111 | 84.63235 294 | 76.1333 3333 | 84.9 |
| INSO | **0.000815737** | 0.798233177 | 10.3066 6667 | 8.284444 444 | 12.27 | 11.734 |
| INS120 | **2.86E-06** | **0.003356934** | 50.6622 2222 | 30.33111 111 | 59.0286 6667 | 27.83866 667 |
| INS180 | **1.30E-06** | **0.02557373** | 39.5722 2222 | 22.83277 778 | 35.0026 6667 | 20.88266 667 |
| INS30 | **0.000414037** | **0.018066406** | 22.1 | 14.49833 333 | 22.598 | 14.20533 333 |
| INS60 | **0.000114652** | **0.004272461** | 40.315 | 23.70222 222 | 45.708 | 23.404 |
| K | 0.665771961 | 0.239135782 | 4.26281 25 | 7.892571 429 | 4.19384 6154 | 4.054285 714 |
| LBP | 0.317407376 | 0.390991211 | 19.5602 7778 | 17.36264 706 | 15.4521 4286 | 17.616 |
| LDL | 0.122171856 | 0.556146527 | 1.15428 5714 | 1.241764 706 | 1.20153 8462 | 1.168571 429 |
| LEPTIN | 0.157752971 | **0.006713867** | 6318.86 6667 | 6480.940 882 | 7200.68 0714 | 4521.537 333 |
| LPA | 0.433978558 | 0.625 | 66.6332 1429 | 32.841 | 303.14 | 214.7509 091 |
| **Lselecti n*** | **0.034002222** | **0.003438** | 1.52277 7778 | 1.686176 471 | 1.59133 3333 | 2.047333 333 |
| Lymph | 0.873712631 | **0.030175493** | 30.1147 0588 | 28.87428 571 | 32.5678 5714 | 28.21428 571 |
| MCP1 | 0.069589183 | **0.006713867** | 245.456 3889 | 256.6388 235 | 211.976 | 252.7773 333 |
| MIP3A | **0.000646785** | 0.100000618 | 20.4654 8387 | 43.76967 742 | 20.3464 2857 | 29.93571 429 |
| monocyt e | 0.810992023 | 0.861220365 | 5.72812 5 | 5.480571 429 | 5.48153 8462 | 5.828571 429 |
| mpo | 0.542937988 | 1 | 366.377 5 | 275.7567 647 | 411.012 6667 | 467.2593 333 |
| Na | 0.097485128 | 0.064957971 | 139.406 25 | 136.8574 286 | 140.307 6923 | 142.2142 857 |
| NEU | 0.279685889 | **0.001656248** | 60.2058 8235 | 58.46857 143 | 52.5057 1429 | 63.59285 714 |
| NGAL | 0.787128593 | 0.276855469 | 129328. 8056 | 125535.0 294 | 137207. 4667 | 174248.3 333 |
| NGF | 0.78915379 | 0.887042291 | 1.8775 | 1.865 | 3.09733 3333 | 2.434 |
| oscatinM | 0.231878957 | 0.454284668 | 105.388 8889 | 86.93939 394 | 85.4333 3333 | 101.1 |
| PAI1 | 0.7103313 | 0.252380371 | 56209.6 6667 | 52769.94 118 | 54084.7 3333 | 61006.73 333 |
| pCPO | **0.003281006** | 0.583007813 | 1264.89 | 1098.934 444 | 1066.73 0667 | 1065.952 857 |
| pCP120 | **3.16E-05** | **0.00012207** | 3983.97 0588 | 2787.309 722 | 3804.86 6667 | 2723.04 |
| pCP180 | **1.50E-06** | **0.024536133** | 3659.41 1765 | 2451.553 333 | 3335.46 6667 | 2526.179 286 |
| pCP30 | **4.56E-05** | **0.013427734** | 1799.17 0286 | 1394.301 389 | 1597.98 0667 | 1362.325 714 |
| pCP60 | **0.000122547** | **0.000610352** | 2780.39 8571 | 1891.040 833 | 2546.80 7333 | 1935.42 |
| PDGFAA | **0.046347912** | 0.488708496 | 5322.73 0571 | 4701.948 824 | 4845.33 3333 | 4507.333 333 |
| PDGFAA BB | **0.004710246** | 0.561401367 | 29869.6 8571 | 24130.63 636 | 26779.7 3333 | 24006.8 |
| **pgh0*** | **0.009070051** | 0.206054688 | 42.2870 5882 | 55.75416 667 | 50.1483 3333 | 53.4025 |
| **pgh0180 *** | **0.002696353** | 0.16015625 | 41.3451 6129 | 60.30111 111 | 46.2636 3636 | 50.87333 333 |
| **pgh120*** | **3.87E-06** | 0.07421875 | 34.2424 2424 | 58.55861 111 | 35.1845 4545 | 42.5275 |
| **pgh30*** | **0.000781945** | 0.233398438 | 46.9967 6471 | 70.15138 889 | 44.2284 6154 | 64.28307 692 |
| **pgh60*** | **8.13E-05** | **0.041311226** | 43.87 | 66.50916 667 | 33.4030 7692 | 56.35083 333 |
| pGLP10 | 0.31839608 | 0.577148438 | 4.09833 3333 | 4.564117 647 | 5.125 | 3.643846 154 |
| pGLP11 20 | 0.066918263 | 0.855224609 | 7.80529 4118 | 10.47416 667 | 8.14666 6667 | 9.07 |
| **pGLP11 80*** | **0.012499022** | 0.501586914 | 5.64781 25 | 7.518333 333 | 6.40066 6667 | 6.457857 143 |
| pGLP13 0 | 0.062569209 | 0.057373047 | 15.2964 7059 | 12.20628 571 | 20.4171 4286 | 11.40714 286 |
| pGLP16 0 | 0.642244034 | 0.807739258 | 14.3494 1176 | 13.20333 333 | 14.4206 6667 | 13.72 |
| pgluc0 | 0.234643616 | 0.637695313 | 29.1234 2857 | 37.29794 118 | 31.7666 6667 | 39.20090 909 |
| **pgluc12 0*** | **0.00154797** | **0.02734375** | 24.0327 2727 | 35.94735 294 | 23.8257 1429 | 29.62545 455 |
| **pgluc18 0*** | **0.000424966** | 0.153576397 | 21.732 | 32.29147 059 | 23.6571 4286 | 30.42818 182 |
| pgluc30 | 0.385465678 | 0.909667969 | 38.742 | 43.28277 778 | 37.3433 3333 | 41.17166 667 |
| pgluc60 | **0.016413683** | 0.518554688 | 35.5568 5714 | 43.96361 111 | 30.278 | 36.68 |
| **pHOMA. IR*** | **0.00102708** | 0.71484375 | 3.95111 0809 | 2.565193 754 | 3.24913 4038 | 2.716974 268 |
| **pins0*** | **0.009625357** | 0.71484375 | 494.009 7143 | 391.0080 556 | 469.047 3333 | 416.3028 571 |
| **pins120*** | **3.16E-05** | 0.057373047 | 2011.77 3529 | 1168.609 722 | 2303.54 2 | 1373.493 077 |
| **pins180*** | **7.24E-06** | 0.067626953 | 1613.70 4118 | 916.6516 667 | 1780.70 1333 | 1152.767 143 |
| **pins30*** | **0.003007197** | 0.172607422 | 817.723 1429 | 561.7958 333 | 780.207 3333 | 639.8921 429 |
| **pins60*** | **0.000283359** | **0.03527832** | 1469.36 6571 | 892.8875 | 1574.94 8667 | 968.9964 286 |
| platelet | 0.964365002 | 0.670003472 | 212.171 4286 | 213.9714 286 | 208.8 | 206.2666 667 |
| PPO | 0.727960433 | **0.049438477** | 85.9882 8571 | 80.31583 333 | 107.546 6667 | 69.67214 286 |
| PP120 | 0.050439826 | **0.03527832** | 231.427 3529 | 258.8033 333 | 203.484 | 260.1364 286 |
| PP180 | 0.446560021 | **0.501586914** | 182.530 8824 | 173.9941 667 | 171.237 3333 | 204.6585 714 |
| PP30 | 0.703771093 | **0.03527832** | 319.300 8571 | 331.6930 556 | 279.108 | 376.6992 857 |
| PP60 | **0.045642266** | **0.016601563** | 305.704 2857 | 330.7513 889 | 237.352 6667 | 318.92 |
| ppyyo | 0.539026541 | 0.91015625 | 42.186 | 49.602 | 51.0175 | 43.56636 364 |
| **ppyy120 *** | **0.038631681** | 0.921875 | 44.3451 6129 | 54.44848 485 | 61.0491 6667 | 54.01538 462 |
| **ppyy180 *** | **0.033681393** | 0.431640625 | 45.3134 4828 | 56.15371 429 | 54.6591 6667 | 51.59230 769 |
| ppyy30 | 0.707845747 | 0.359375 | 56.9793 3333 | 55.73625 | 77.2830 7692 | 64.33909 091 |
| ppyy60 | 0.33367527 | 0.764648438 | 56.1743 75 | 59.37029 412 | 69.9369 2308 | 56.77923 077 |
| RANTES | 0.179124002 | 0.524475098 | 58731.5 | 50811.91 176 | 50815.2 | 47562.53 333 |
| RBC | 0.321296464 | 0.306429005 | 4.78342 8571 | 4.832 | 5.008 | 4.945333 333 |
| RCv | | 0.942925705 | 9.93617 6471 | 2.580285 714 | 9.274 | 0.442142 857 |
| resistin | 0.624272656 | 0.276855469 | 15960.5 | 14639.64 706 | 21493.7 3333 | 25013.66 667 |
| **rGT*** | **5.29E-05** | **0.020263672** | 42.7571 4286 | 24.82285 714 | 60.0733 3333 | 43.12142 857 |
| SBP | 0.122812744 | 0.064531987 | 127.027 7778 | 120.8611 111 | 127.8 | 119.8 |
| **sicam1*** | **2.21E-06** | **0.018066406** | 167.553 3333 | 112.2894 118 | 163.149 3333 | 122.9993 333 |
| **Spselect in*** | **0.001039933** | 0.120544434 | 142.373 3333 | 92.83058 824 | 139.506 | 102.7933 333 |
| **svcam1*** | **0.000430483** | **0.041259766** | 674.316 9444 | 484.8473 529 | 738.345 3333 | 545.498 |
| TBIL | 0.316992017 | 0.216308594 | 15.3617 6471 | 16.35714 286 | 13.7428 5714 | 14.9 |
| TC | 0.059412126 | **0.026855469** | 4.96885 7143 | 4.735588 235 | 5.51461 5385 | 4.882857 143 |
| **TG*** | **0.000665817** | **0.01668677** | 2.48285 7143 | 1.525 | 2.53230 7692 | 1.693571 429 |
| TNFa | 0.877708881 | 0.07823218 | 4.66083 3333 | 4.772647 059 | 3.96266 6667 | 4.572 |
| TNFBM | 0.572530012 | 0.900061308 | 35.625 | 38.44117 647 | 45.3333 3333 | 44.7 |
| **TPRO*** | **0.031620353** | 0.57587042 | 72.0794 1176 | 70.11428 571 | 74.0857 1429 | 73.55 |
| Trophoni nM | 0.796523868 | 0.167835191 | 17.4861 1111 | 16.15151 515 | 16.5333 3333 | 17.9 |
| urea | 0.205820381 | 0.700616425 | 4.89484 8485 | 4.731428 571 | 5.08133 3333 | 4.953846 154 |
| vegfa | 0.253948028 | 0.172607422 | 516.596 7647 | 475.2033 333 | 308.431 4286 | 340.1686 667 |
| **vegfc** | **0.025448661** | 0.414306641 | 121.530 8824 | 106.3214 706 | 113.845 3846 | 128.6914 286 |
| **vegfd** | **9.05E-06** | **0.010742188** | 193.542 7778 | 145.3523 529 | 249.552 1429 | 195.0753 333 |
| **WAIST*** | **0.004813593** | **0.029960994** | 91.1142 8571 | 88.45277 778 | 92.2666 6667 | 88.92857 143 |
| WBC | 0.387914524 | 0.334179383 | 6.41342 8571 | 6.912571 429 | 6.38 | 6.146 |
| **WHR*** | **0.036332936** | 0.068270928 | 0.90971 4286 | 0.895555 556 | 0.94 | 0.913571 429 |

## Claims

1. A use of characteristics of gut microbiota metagenome as a screening marker of Acarbose efficacy in patients with Type 2 diabetes, wherein the characteristic of gut microbiota metagenome is Bacteroides enterotype.

2. The use according to claim 1, wherein the Bacteroides enterotype is determined by DNA sequencing or PCR amplification of gut microbiota in feces in vitro.

3. The use according to claim 2, wherein the PCR amplification specifically comprises: extract the DNA of gut microbiota in feces in vitro and perform 16Srna PCR amplification for specific enrichment strains.

4. The use according to claim 1, wherein the Bacteroides enterotype is determined by detecting secondary bile acid in the in vitro blood samples.

5. The use according to claim 4, wherein the detection of secondary bile acid comprises the following steps:
S1. Sample pretreatment: Add 300 µL of internal standard methanol to every 75 µL of blood samples, to extract the target compound and precipitate the protein, vortex, centrifuge and draw the supernatant, then lyophilize, re-dissolve in 50 µL of acetonitrile solution (25%, volume), and wait for sample injection;
S2. Detection: conduct sample analysis using 1290 Infinity liquid phase and 6460A triple quadrupole mass spectrometry system;
Perform the liquid phase separation using 100mm×2.1mm ACQUITY UPLC C8 column having a particle size of 1.7µm, of which, phase A is 10 mM NH4HCO3 aqueous solution, phase B is pure acetonitrile; initially 25% phase B (by volume), retaining 0.5 min, followed by increased to 40% phase B (by volume) linearly within 12.5 min, then increased to 90% (by volume) within 1 min, flush the system for 3min, recover to 25% phase B (by volume) in 0.5 min, after equilibrating 2.5 min, the flow rate is 0.35 ml/min, column temperature is 35°C and the injection volume is 5 µL;
Mass spectrometry is performed by ESI source negative ion mode, with main parameters as follows: Gas Temp: 350°C; Gas Flow: 8 I/min; Nebulizer: 40 psi; Sheath Gas Temp: 400 °C; Sheath Gas Flow: 8 I/min; Capillary: 3500 V; Nozzle voltage: 400 V.

6. The use according to claim 1, wherein the efficacy of Acarbose in the patients with Type 2 diabetes and Bacteroides enterotype includes improving the insulin resistance, reducing the secondary bile acid, and promoting the reduction of cardiovascular risks in addition to glucose-lowering.

7. The use according to claim 6, wherein the indicators for reducing the secondary bile acid include GDCA, TDCA, TLCA, and the indicators for reducing the taurine-conjugated bile acid include TCA, TDCA, TLCA, TUDCA.

8. The use according to claim 6, wherein the indicators for improving insulin resistance include decreased fasting blood glucose, decreased fasting C peptide and insulin level, down-regulated waist-to-hip ratio, down-regulated HOMA insulin resistance index and up-regulated Adiponectin.

9. The use according to claim 6, wherein the indicators that promote the reduction of cardiovascular risks include decreased PDGFAA, PDGFAABB, endothelin, and VegfC plasma factors.

## Patentansprüche

1. Verwendung der Charakteristiken des Metagenoms der Darmmikrobiota als einem Screeningmarker der Wirksamkeit von Acarbose bei Patienten mit Typ 2-Diabetes, wobei die Charakteristik des Metagenoms der Darmmikrobiota der Bakteroides-Enterotyp ist.

2. Verwendung gemäß Anspruch 1, wobei der Bakteroides-Enterotyp durch DNA-Sequenzierung oder PCR-Amplifikation der Darmmikrobiota im Stuhl in vitro bestimmt wird.

3. Verwendung gemäß Anspruch 2, wobei die PCR-Amplifikation spezifisch umfasst: Extrahieren der DNA der Darmmikrobiota im Stuhl in vitro und Durchführen einer 16sRNA PCR-Amplifikation für spezifische Anreicherungsstämme.

4. Verwendung gemäß Anspruch 1, wobei der Bakteroides-Enterotyp durch Nachweisen von sekundärer Gallensäure in den in vitro-Blutproben bestimmt wird.

5. Verwendung gemäß Anspruch 4, wobei der Nachweis der sekundären Gallensäure die folgenden Schritte umfasst:
S1. Probenvorbehandlung: Zugeben von 300 µl Methanol als internem Standard zu jeweils 75 µl der Blutproben, um die Zielverbindung zu extrahieren und das Protein auszufällen, Vortexen, Zentrifugieren und Absaugen des Überstands, dann Lyophilisieren, Wiederauflösen in 50 µl Acetonitrillösung (25 %, Volumen) und Warten für die Probeninjektion;
S2. Detektion: Durchführen der Probenanalyse unter Verwendung des 1290 Infinity Flüssigphasen- und 6460A Triple-Quadrupol-Massenspektrometriesystems;
Durchführen der Flüssigphasentrennung unter Verwendung einer 100mm x 2,1mm ACQUITY UPLC C8-Säule mit einer Partikelgröße von 1,7 µm, wovon Phase A eine wässrige Lösung von 10 mM NH4HCO3 ist, Phase B reines Acetonitril ist; initial 25 % Phase B (nach Volumen), rückgehalten 0,5 min, gefolgt von Erhöhung auf 40 % Phase B (nach Volumen), linear innerhalb von 12,5 min, dann Erhöhung auf 90 % (nach Volumen) innerhalb von 1 min, Spülen des Systems für 3 min, Wiederherstellen von 25 % Phase B (nach Volumen) in 0,5 min, nach Äquilibrieren für 2,5 min ist die Flussrate 0,35 ml/min, ist die Säulentemperatur 35 °C und ist das Injektionsvolumen 5 µl;
Massenspektrometrie wird im Negativ-Modus der ESI-Ionenquelle, mit den folgenden Hauptparametern durchgeführt: Gastemp: 350 °C; Gasstrom: 8 l/min; Zerstäuber: 40 psi; Mantelgastemp: 400 °C; Mantelgasstrom: 8 I/min; Kapillare: 3500 V; Düsenspannung: 400 V.

6. Verwendung gemäß Anspruch 1, wobei die Wirksamkeit der Acarbose bei den Patienten mit Typ 2-Diabetes und Bakteroides-Enterotyp das Verbessern der Insulinresistenz, Verringern der sekundären Gallensäure und Fördern der Verminderung von kardiovaskulären Risiken über die Glukosesenkung hinaus umfasst.

7. Verwendung gemäß Anspruch 6, wobei die Indikatoren für die Verringerung der sekundären Gallensäure GDCA, TDCA, TLCA umfassen und die Indikatoren für die Verringerung der Taurin-konjugierten Gallensäure TCA, TDCA, TLCA, TUDCA umfassen.

8. Verwendung gemäß Anspruch 6, wobei die Indikatoren für die Verbesserung der Insulinresistenz verringerte Fasten-Blutglukose, verringerter Fasten-C-Peptid- und Insulinspiegel, herabreguliertes Taille-Hüfte-Verhältnis, herabregulierter HOMA-Insulinresistenz-Index und heraufreguliertes Adiponektin umfassen.

9. Verwendung gemäß Anspruch 6, wobei die Indikatoren, welche die Verminderung der kardiovaskulären Risiken fördern, verringerte PDGFAA-, PDGFAABB-, Endothelin- und VEGF-C-Plasmafaktoren umfassen.

## Revendications

1. Utilisation de caractéristiques du métagénome du microbiote intestinal en tant que marqueur de dépistage de l'efficacité de l'acarbose chez les patients atteints de diabète de type 2, dans laquelle la caractéristique du métagénome du microbiote intestinal est un entérotype Bacteroides.

2. Utilisation selon la revendication 1, dans laquelle l'entérotype Bacteroides est déterminé par un séquençage d'ADN ou une amplification par PCR du microbiote intestinal dans des matières fécales in vitro.

3. Utilisation selon la revendication 2, dans laquelle l'amplification par PCR comprend spécifiquement : l'extraction de l'ADN de microbiote intestinal dans des matières fécales in vitro et la réalisation d'une amplification par PCR de 16Srna pour les souches d'enrichissement spécifiques.

4. Utilisation selon la revendication 1, dans laquelle l'entérotype Bacteroides est déterminé par détection d'acide biliaire secondaire dans les échantillons de sang in vitro.

5. Utilisation selon la revendication 4, dans laquelle la détection d'acide biliaire secondaire comprend les étapes suivantes :
S1. prétraitement de l'échantillon : ajout de 300 µl d'étalon interne méthanol à chaque 75 µl; d'échantillons de sang, pour extraire le composé cible et précipiter la protéine, soumission à un vortex, centrifugation et aspiration du surnageant, puis lyophilisation, re-dissolution dans 50 µl; de solution d'acétonitrile (25 %, volume), et attente de l'injection d'échantillon ;
S2. détection : réalisation d'une analyse d'échantillon en utilisant un 1290 Infinity en phase liquide et un système de spectrométrie de masse triple quadripôle 6460A ;
réalisation de la séparation de phase liquide en utilisant une colonne ACQUITY UPLC C8 100 mm x 2,1 mm ayant une taille de particules de 1,7 µm, dont la phase A est une solution aqueuse de NH4HCO3 10 mM, la phase B est de l'acétone pur ; initialement 25 % de la phase B (en volume), rétention pendant 0,5 min, suivie par une augmentation jusqu'à 40 % de phase B (en volume) de manière linéaire en 12,5 min, puis augmentation jusqu'à 90 % (en volume) en 1 minute, rinçage du système pendant 3 min, récupération jusqu'à 25 % de phase B (en volume) en 0,5 min, après équilibrage pendant 2,5 min, le débit est de 0,35 ml/min, la température de colonne est de 35 °C et le volume d'injection est de 5 µl ;
la spectrométrie de masse est réalisée par une source ESI en mode ions négatifs, avec les paramètres principaux définis ainsi : température du gaz : 350 °C ; débit de gaz : 8 l/min ; nébuliseur : 40 psi ; température du gaz de gaine : 400 °C; débit du gaz de gaine : 8 l/ min ; capillaire : 3500 V; tension de buse : 400 V.

6. Utilisation selon la revendication 1, dans laquelle l'efficacité de l'acarbose chez les patients présentant un diabète de type 2 et un entérotype Bacteroides inclut l'amélioration de la résistance à l'insuline, la réduction de l'acide biliaire secondaire, et la promotion de la réduction des risques cardiovasculaires outre une réduction du glucose.

7. Utilisation selon la revendication 6, dans laquelle les indicateurs de réduction de l'acide biliaire secondaire incluent les GDCA, TDCA, TLCA, et les indicateurs de réduction de l'acide biliaire conjugués à la taurine incluent les TCA, TDCA, TLCA, TUDCA.

8. Utilisation selon la revendication 6, dans laquelle les indicateurs d'amélioration de la résistance à l'insuline comprennent une glycémie à jeun réduite, un taux de peptide C et d'insuline à jeun réduit, un rapport taille/hanches régulé à la baisse, un indice de résistance à l'insuline HOMA régulé à la baisse et l'adiponectine régulée à la hausse.

9. Utilisation selon la revendication 6, dans laquelle les indicateurs qui promeuvent la réduction des risques cardiovasculaires incluent des facteurs plasmatiques PDGFAA, PDGFAABB, endothéline et VegfC réduits.
